# EUROPEAN PATENT SPECIFICATION

(11) **EP 1 408 879 B1**
(45) Date of publication and mention of the grant of the patent: **28.09.2005**
(21) Application number: 02747751.2
(22) Date of filing: 19.07.2002
(51) Int. Cl.: A61F 2/06, C08F 2/50, C09D 133/06

(54) **METHOD FOR THE MANUFACTURE OF A PLASTIC FILM MATERIAL SUITABLE FOR IMPLANTATION, AND STENT FORMED USING IT**
VERFAHREN ZUR HERSTELLUNG VON KUNSTSTOFFFOLIENMATERIAL GEEIGNET FÜR IMPLANTATION UND DARAUS HERGESTELLTER STENT
PROCEDE DE FABRICATION DE FILM PLASTIQUE POUR IMPLANTATION ET ENDOPROTHESE FABRIQUE A BASE DE CE FILM

(30) Priority: 24.07.2001 NL 1018619
(43) Date of publication of application: 21.04.2004
(73) Proprietor: Rigitec B.V., 7535 PG Enschede (NL)
(72) Inventor: GLASTRA, Hendrik, NL-7535 PG Enschede (NL); DE KLEIJN, Paul, NL-3781 EZ Voorthuizen (NL)
(74) Representative: Timmers, Cornelis Herman Johannes
(86) International application number: PCT/NL2002/000490
(87) International publication number: WO 2003/009782

(56) References cited:
- EP-A- 0 287 516
- WO-A-01/26582
- WO-A-96/09086
- AT-B- 407 479
- US-A- 4 048 034

## Description

The invention relates to a method for the manufacture of a plastic film material suitable for implantation, by providing a pharmaceutically acceptable, flexible plastic carrier material with a layer of a photopolymerizable synthetic resin composition containing a photo-initiator.

Such a plastic film material, in the form of an adhesive composition, is suggested in European Patent Application No. 0 617 930. This known material is contained in a double-walled case, however, when it is used as a stent. The external diameter of a stent must not be too large, since the stent has to be inserted into a vein and serves to strengthen weak spots in the wall of the vein; nor, in order to maintain adequate circulation through the blood vessel, may the internal diameter of the stent after application in the vein be too small.

Another requirement to be satisfied by a stent is that it must have a certain degree of flexibility in a longitudinal direction in order to imitate the natural state of a vein as well as possible. It is desirable, therefore, that the material of which the stent is made should have a small thickness, preferably up to 15 µm, but while retaining strength and flexibility in a longitudinal direction.

In order to facilitate the placing of a stent in a vein, the film material of the stent is rolled up (as is illustrated in Fig. 3 of EP-A-0 617 930) and the rolled-up stent is brought to the desired place in a vein with the aid of the catheter, extended or unrolled with the aid of a balloon incorporated in the catheter, and then cured by exposure to light.

An acrylate resin composition is usually employed as the photopolymerizable composition. In an uncured state, however, such a resin composition has such a tack that use thereof is possible only when the composition is incorporated as a sandwich between other layers, as is the case with the case body according to EP-A-0 617 930.

With the aim of making the thickness of the stent smaller, therefore, a photopolymerizable synthetic resin composition would be desirable which renders the presence of a case body unnecessary and yet can still be rolled up and unrolled, in an uncured state, without changing the thickness of the composition.

A method has now been found for the manufacture of a plastic film material suitable for implantation, of the type mentioned in the preamble,, which is characterized in that the synthetic resin composition is provided with a tack-reducing synthetic resin component. An example of such a component is polyurethane.

Because of the use of the plastic film material in question as implantation material, it is essential that no substances poisonous to the body, originating from the polymerization initiator, are released during curing of the material. It has now been found that this can be prevented by using photo-initiators which are themselves already a polymer. An example of such an initiator is the oligomer of 2-hydroxy-2-methyl-1-[4-(1-methylvinyl)-phenyl]-propanone, also known as Esacure kip 150, from the company Lamberti SpA. This initiator, however, requires a fairly long exposure time of usually about 2 min, which is actually too long under certain conditions.

Accelerated curing can be achieved by adding an accelerator such as a tertiary amine having α-H atoms.

A problem with such accelerators, however, is that they have to be added at such a high concentration that they can exhibit cytotoxic properties.

It has now been found that the problem of the formation of poisonous degradation products from the accelerator, and the problem of the long exposure time, can be prevented by using an initiator which generates its own curing accelerator after activation. In this way it is possible to achieve complete curing of the synthetic resin composition within 10 sec.

The initiator according to the invention is preferably an α-aminoacetophenone derivative with formula I: 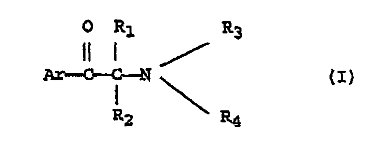 in which Ar represents C₆-C₁₄ aryl groups which are unsubstituted or substituted by one or more halogen, hydroxyl, C₁-C₄ alkyl, C₁-C₄ alkoxy, C₁-C₄ alkylthio, C₁-C₄ alkylamino, or C₁-C₄ dialkylamino or N-morpholino groups, and R₁ and R₂ independently of each other are hydrogen, C₁-C₈ alkyl, C₅-C₈ cycloalkyl, C₇-C₉ phenylalkyl, and R₃ and R₄ independently of each other are hydrogen, a C₁-C₈ alkyl, C₅-C₈ cycloalkyl or C₇-C₉ phenylalkyl, which groups may be substituted by C₁-C₄ alkoxy groups.

Preferred initiators are the following compounds with formula I, in which Ar represents a p-N-morpholinophenyl group or p-methylthiophenyl group, R₁ and R₂ independently of each other represent a C₁-C₄ alkyl or benzyl group, and R₃ and R₄ each represent methyl or, together with the nitrogen atom with which they are linked, an N-morpholino group.

Such initiators are available commercially as for example Irgacure 907 and Irgacure 369, from the company Ciba-Geigy AG.

It is pointed out that such polymerization initiators are known per se from European Patent Application No. 0 287 516. According to this publication, however, a photosensitizer is always needed in addition to the photopolymerization initiator. Moreover, only the application in blister packs is named as an application of films of the polymers formed in the medical or pharmaceutical field.

This publication does not contain any mention of the production of a composition which is suitable for application in the manufacture of a stent.

It is observed that stents of which the body is made of a thin plastics film material are known in itself, as disclosed in WO 01/26582. The specific stent which is subject of this publication comprises a tubular body part made of a material which shrinks at temperatures higher than the human body temperature; it is to be positioned into a blood vessel in this shrunken state. When exposed to, and thus cooled to, the body temperature the stent expands to the desired, greater, diameter. The specific method which is subject of the present invention is not disclosed in, and cannot be derived from, this publication.

Another aspect of the present invention is the fact that a monomer composition is formed consisting of a mixture of an epoxy acrylate, an aliphatic polyester acrylate and a photo-initiator having formula I, as described above, this mixture is mixed with a polyurethane solution in dimethylacetamide, to form the synthetic resin composition, this composition is applied to a flexible plastic carrier material and the solvent is removed in a manner known per se.

Any carrier material which can be formed into a flexible film and which is pharmaceutically acceptable can be used as the plastic carrier material. A carrier which has been manufactured as a gauze is preferably used; the preferred material is nylon 6.6, although other plastics can also be used. The gauze suitably has a distance between the fibres of 40-100 mµ, preferably 60 mµ, and a fibre thickness of 20-50 mµ, preferably about 30 mµ. An example of this is a fine silkscreen sieve material, which is commonly used in bandaging.

The invention also relates to an expandable intravascular stent, which is obtained using a plastic film material according to the invention, as described above.

The invention is illustrated in greater detail by means of the following examples.

### EXAMPLE 1

A monomer mixture was formed, consisting of the following components:
a) 150 parts by weight of epoxy acrylate (Actilane 320GP30, AKZO Nobel)
b) 10 parts by weight of photo-initiator (Irgacure 369, Ciba-Geigy AG), and
c) 150 parts by weight of dipentaerythritol pentaacrylate (Photocure SR 399, Cray Valley) by dissolving component b) in component a), with slight heating (to about 80°C). Component c) was then incorporated into the still warm mixture, while stirring. After the whole mixture formed a homogeneous whole, it was ready for manufacture of a stent.

### EXAMPLE 2

40 parts by weight of the mixture prepared in Example 1 was thoroughly mixed with 30 parts by weight of a polyurethane solution in dimethylacetamide (Chronoflex AR, a product consisting of 60% by weight polyurethane/40% acetamide, available commercially from Cardio Tech. Int. Inc., USA) and was applied with the aid of a doctor blade to a nylon 6.6 gauze commonly used in bandaging, in a quantity of 7 g of resin mixture to 3 g of gauze material.

The gauze material so coated and impregnated was cut into portions of 10 x 19 mm, and these portions were wrapped on a rod with a diameter of 3 mm, forming cases. These cases were dried in a vacuum oven at 80°C for 5 min, and then for some time at 140°C and under vacuum until all the dimethylacetamide had disappeared.

In this way, UV curable stents of 3 x 10 mm were obtained which could easily be rolled up and unrolled again without damage to the synthetic resin layer applied. Curing of the synthetic resin layer was achieved within 2 sec by exposure to a UV lamp.

## Claims

1. Method for the manufacture of a plastic film material suitable for implantation, by providing a pharmaceutically acceptable, flexible plastic carrier material with a layer of a photopolymerizable synthetic resin composition containing a photo-initiator, **characterized in that** the synthetic resin composition is given a tack-reducing component.

2. Method according to claim 1, **characterized in that** the tack-reducing component is chosen from polyurethane, a second initiator or a mixture thereof.

3. Method according to claim 2, **characterized in that** the initiator generates a curing accelerator after activation.

4. Method according to claims 1 to 3, **characterized in that** the initiator is chosen from an oligomer of 2-hydroxy-2-methyl-1-[4-(1-methylvinyl)-phenyl]-propanone, or an α-aminoacetophenone derivative.

5. Method according to claims 1 to 4, **characterized in that** the initiator is an α-aminoacetophenone derivative, with formula (I) : 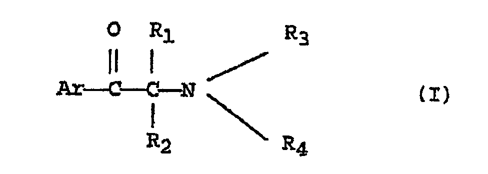 in which Ar represents C₆-C₁₄ aryl groups which are unsubstituted or substituted by one or more halogen, hydroxyl, C₁-C₄ alkyl, C₁-C₄ alkoxy, C₁-C₄ alkylthio, C₁-C₄ alkylamino, or C₁-C₄ dialkylamino or N-morpholino groups, and R₁ and R₂ independently of each other are hydrogen, C₁-C₈ alkyl, C₅-C₈ cycloalkyl, C₇-C₉ phenylalkyl, and R₃ and R₄ independently of each other are hydrogen, a C₁-C₈ alkyl, C₅-C₈ cycloalkyl or C₇-C₉ phenylalkyl, which groups may be substituted by C₁-C₄ alkoxy groups.

6. Method according to claim 5, **characterized in that** the initiator is an α-aminoacetophenone derivative with formula (I), in which Ar represents a p-N-morpholinophenyl group or p-methylthiophenyl group, R₁ and R₂ independently of each other represent a C₁-C₄ alkyl or benzyl group, and R₃ and R₄ each represent methyl or, together with the nitrogen atom with which they are linked, an N-morpholino group.

7. Method according to claims 1 to 6, **characterized in that** a synthetic resin composition is formed from a mixture of an epoxy acrylate, an aliphatic polyester acrylate and a photopolymerization initiator with formula (I): 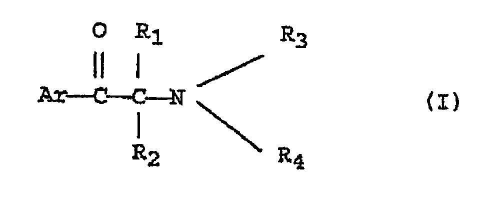 in which Ar represents C₆-C₁₄ aryl groups which are unsubstituted or substituted by one or more halogen, hydroxyl, C₁-C₄ alkyl, C₁-C₄ alkoxy, C₁-C₄ alkylthio, C₁-C₄ alkylamino, or C₁-C₄ dialkylamino or N-morpholino groups, and R₁ and R₂ independently of each other are hydrogen, C₁-C₈ alkyl, C₅-C₈ cycloalkyl, C₇-C₉ phenylalkyl, and R₃ and R₄ independently of each other are hydrogen, a C₁-C₈ alkyl, C₅-C₈ cycloalkyl or C₇-C₉ phenylalkyl, which groups may be substituted by C₁-C₄ alkoxy groups, this mixture is mixed with a polyurethane solution in acetamide, forming a synthetic resin composition, this composition is applied to a flexible plastic carrier material and the acetamide is removed in a manner known per se.

8. Method according to claims 1 to 7, **characterized in that** the plastic carrier material is a gauze of nylon 6.6 with a distance between the fibres of 40-100 mµ, preferably 60 mµ, and a fibre thickness of 20-50 mµ, preferably about 30 mµ.

## Patentansprüche

1. Verfahren für die Herstellung eines Folienmaterials aus Plastik für die Implantation, durch Bereitstellung eines pharmazeutisch akzeptierbaren, flexiblen Trägermaterials aus Plastik mit einer Schicht einer photopolymerisierbaren synthetischen Harzverbindung, die einen Photoinitiator enthält, **dadurch gekennzeichnet, dass** die synthetische Harzverbindung eine die Haftung reduzierende Komponente enthält.

2. Verfahren gemäß Anspruch 1, **dadurch gekennzeichnet, dass** die die Haftung reduzierende Komponente ausgewählt ist aus Polyurethan, einem zweiten Inhibitor oder einem Gemisch aus den vorgenannten Substanzen.

3. Verfahren gemäß Anspruch 2, **dadurch gekennzeichnet, dass** der Initiator nach Aktivierung einen Aushärtungsbeschleuniger ausbildet.

4. Verfahren gemäß einem der Ansprüche 1 - 3, **dadurch gekennzeichnet, dass** der Initiator ausgewählt ist aus einem Oligomer des 2-Hydroxy-2-Methyl-1-[4-(1-Methylvinyl)-Phenyl]-Propanon oder einem α-Aminoacetophenon-Derivat.

5. Verfahren gemäß einem der Ansprüche 1 - 3, **dadurch gekennzeichnet, dass** der Initiator ein α-Aminoacetophenon-Derivat mit der Formel (I) : 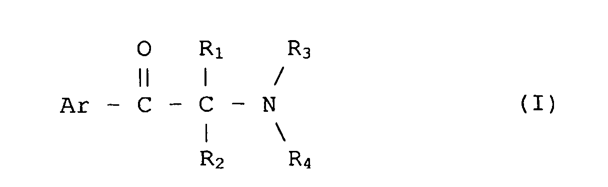 ist, in welcher Ar C₆-C₁₄ Arylgruppen darstellt, die unsubstituiert oder mit einem oder mehreren Halogenen, einer oder mehreren Hydroxylgruppen, C₁-C₄ Alkylgruppen, C₁-C₄ Alkoxygruppen, C₁-C₄ Alkylthiogruppen, C₁-C₄ Alkylaminogruppen oder C₁-C₄ Dialkylamino- oder N-morpholinogruppen substituiert sind, und in welcher R₁ und R₂ unabhängig voneinander Wasserstoff, eine C₁-C₈ Alkylgruppe, C₅-C₈ Cycloalkylgruppe oder C₇-C₉ Phenylalkylgruppe sind, und in welcher R₃ und R₄ unabhängig voneinander Wasserstoff, eine C₁-C₈ Alkylgruppe, C₅-C₈ Cycloalkylgruppe oder C₇-C₉ Phenylalkylgruppe sind, wobei diese Gruppen durch C₁-C₄ Alkoxygruppen substituiert werden können.

6. Verfahren gemäß Anspruch 5, **dadurch gekennzeichnet, dass** der Initiator ein α-Aminoacetophenon-Derivat der Formel (I) ist, in welcher Ar eine p-N-Morpholinophenylgruppe oder eine p-Methylthiophenylgruppe darstellt,
und in welcher R₁ und R₂ unabhängig voneinander eine C₁-C₄ Alkyl- oder Benzylgruppe sind,
und in welcher R₃ und R₄ jeweils Methyl oder, zusammen mit dem Stickstoffatom, an das sie gebunden sind, eine N-Morpholinogruppe darstellen.

7. Verfahren gemäß einem der Ansprüche 1 - 6, **dadurch gekennzeichnet, dass** aus einem Gemisch aus einem Epoxyacrylat, einem aliphatischen Polyesteracrylat und einem Photopolymerisationsinitiator mit der Formel (I) : 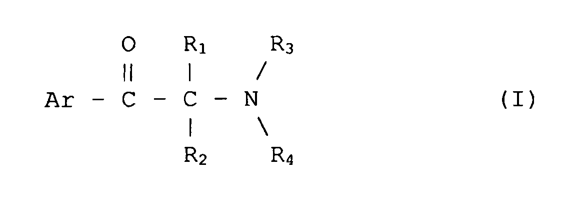 eine synthetische Harzverbindung gebildet wird, in welcher Ar C₆-C₁₄ Arylgruppen darstellt, die unsubstituiert oder mit einem oder mehreren Halogenen, einer oder mehreren Hydroxylgruppen, C₁-C₄ Alkylgruppen, C₁-C₄ Alkoxygruppen, C₁-C₄ Alkylthiogruppen, C₁-C₄ Alkylaminogruppen oder C₁-C₄ Dialkylamino- oder N-morpholinogruppen substituiert sind,
und in welcher R₁ und R₂ unabhängig voneinander Wasserstoff, eine C₁-C₈ Alkylgruppe, C₅-C₈ Cycloalkylgruppe oder C₇-C₉ Phenylalkylgruppe sind, und in welcher R₃ und R₄ unabhängig voneinander Wasserstoff, eine C₁-C₈ Alkylgruppe, C₅-C₈ Cycloalkylgruppe oder C₇-C₉ Phenylalkylgruppe sind,
wobei diese Gruppen durch C₁-C₄ Alkoxygruppen substituiert werden können, und wobei dieses Gemisch mit einer Polyurethan-Acetamid-Lösung vermischt wird, wodurch eine synthetische Harzverbindung gebildet wird,
diese Verbindung auf ein flexibles Trägermaterial aus Plastik aufgetragen wird
und das Acetamid auf eine an sich bekannte Weise entfernt wird.

8. Verfahren gemäß einem der Ansprüche 1 - 7, **dadurch gekennzeichnet, dass** das Trägermaterial aus Plastik eine Gaze aus Nylon 6.6 mit einem Faserabstand von 40 - 100 µm, bevorzugt 60 µm, und einer Faserdicke von 20 - 50 µm, bevorzugt ungefähr 30 mm, ist.

## Revendications

1. Procédé de fabrication d'un matériau de film plastique adapté à une implantation consistant à fournir à un matériau de support en plastique flexible pharmaceutiquement acceptable d'une couche d'une composition de résine synthétique photopolymérisable contenant un photo-initiateur, **caractérisé en ce que** la composition de résine synthétique reçoit un composant réduisant l'adhérence.

2. Procédé selon la revendication 1, **caractérisé en ce que** le composant réduisant l'adhérence est choisi parmi le polyuréthane, un second initiateur ou un mélange de ceux-ci.

3. Procédé selon la revendication 2, **caractérisé en ce que** l'initiateur génère un accélérateur de traitement après activation.

4. Procédé selon les revendications 1 à 3, **caractérisé en ce que** l'initiateur est choisi parmi un oligomère de 2-hydroxy-2-méthyl-1-[4-(1-méthylvinyl)-phényl]-propanone ou un dérivé d'α-aminoacétophénone.

5. Procédé selon les revendications 1 à 4, **caractérisé en ce que** l'initiateur est un dérivé d'α-aminoacétophénone de formule (I) : 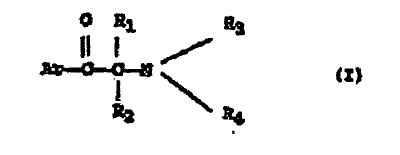 dans laquelle Ar représente un groupe aryle en C₆-C₁₄ qui est non substitué ou substitué par un ou plusieurs halogènes, groupes hydroxyle, alkyle en C₁-C₄, alcoxy en C₁-C₄, (alkyl en C₁-C₄) thio, (alkyl en C₁-C₄)amino, (dialkyl en C₁-C₄)amino ou N-morpholino, et R₁ et R₂, indépendamment l'un de l'autre, représentent un hydrogène, un groupe alkyle en C₁-C₈, cycloalkyle en C₅-C₈ ou phénylalkyle en C₇-C₉, et R₃ et R₄, indépendamment l'un de l'autre, représentent un hydrogène, un groupe alkyle en C₁-C₈, cycloalkyle en C₅-C₈ ou phénylalkyle en C₇-C₉, lesquels groupes pouvant être substitués par des groupes alcoxy en C₁-C₄.

6. Procédé selon la revendication 5, **caractérisé en ce que** l'initiateur est un dérivé d'α-aminoacétophénone de formule (I) dans laquelle Ar représente un groupe p-N-morpholinophényle ou un groupe p-méthylthiophényle, R₁ et R₂, indépendamment l'un de l'autre, représentent un groupe alkyle en C₁-C₄ ou benzyle, et R₃ et R₄ représentent chacun un groupe méthyle ou, conjointement avec l'atome d'azote auquel ils sont liés, un groupe N-morpholino.

7. Procédé selon les revendications 1 à 6, **caractérisé en ce qu'**une composition de résine synthétique est formée à partir d'un mélange d'un acrylate d'époxyde, d'un acrylate de polyester aliphatique et d'un initiateur de photopolymérisation de formule (I) : 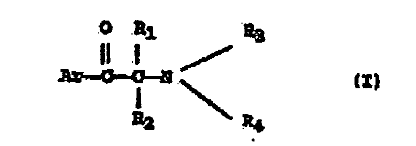 dans laquelle Ar représente un groupe aryle en C₆-C₁₄ qui est non substitué ou substitué par un ou plusieurs halogènes, groupes hydroxyle, alkyle en C₁-C₄, alcoxy en C₁-C₄, (alkyl en C₁-C₄) thio, (alkyl en C₁-C₄) amino, (dialkyl en C₁-C₄)amino ou N-morpholino, et R₁ et R₂, indépendamment l'un de l'autre, représentent un hydrogène, un groupe alkyle en C₁-C₈, cycloalkyle en C₅-C₈ ou phénylalkyle en C₇-C₉, et R₃ et R₄, indépendamment l'un de l'autre, représentent un hydrogène, un groupe alkyle en C₁-C₈, cycloalkyle en C₅-C₈ ou phénylalkyle en C₇-C₉, lesquels groupes pouvant être substitués par des groupes alcoxy en C₁-C₄, ce mélange étant mélangé avec une solution de polyuréthane dans de l'acétamide, formant une composition de résine synthétique, cette composition étant appliquée à un matériau de support en plastique flexible et l'acétamide étant éliminé d'une manière connue par elle-même.

8. Procédé selon les revendications 1 à 7, **caractérisé en ce que** le matériau de support en plastique est une gaze de nylon 6,6 avec une distance entre les fibres de 40-100 µm, de préférence 60 µm, et une épaisseur de fibre de 20-50 µm, de préférence environ 30 µm.
